Europäisches Patentamt

(19) European Patent Office

Office européen des brevets

(11) Publication number: **0 040 899**
**B1**

(12) **EUROPEAN PATENT SPECIFICATION**

(45) Date of publication of patent specification: **12.09.84**

(51) Int. Cl.³: **A 61 K 9/46,** A 61 K 9/22

(21) Application number: **81300572.5**

(22) Date of filing: **12.02.81**

(54) **Method of delivering drug with aid of effervescent activity generated in environment of use.**

(30) Priority: **25.04.80 US 143644**

(43) Date of publication of application:
**02.12.81 Bulletin 81/48**

(45) Publication of the grant of the patent:
**12.09.84 Bulletin 84/37**

(84) Designated Contracting States:
**AT BE CH DE FR GB IT LI NL SE**

(56) References cited:
**BE-A- 676 458**
**FR-A-2 323 440**
**FR-A-2 386 316**

(73) Proprietor: **ALZA CORPORATION**
**950 Page Mill Road**
**Palo Alto, California 94304 (US)**

(72) Inventor: **Bonsen, Pieter**
**237 Avalon Dr.**
**Los Altos California (US)**
Inventor: **Wong, Patrick Seck-Lai**
**37 Mody 4th Floor**
**Kowloon (HK)**
Inventor: **Theeuwes, Felix**
**1643 Fallen Leaf Lane**
**Los Altos California (US)**

(74) Representative: **Evans, David Charles et al**
**F.J. CLEVELAND & COMPANY 40-43, Chancery**
**Lane**
**London, WC2A 1JQ (GB)**

Courier Press, Leamington Spa, England.

EP 0 040 899 B1

## Description

### Field of the Invention

This invention relates to an osmotically driven device for delivering an active agent, such as a drug, to a fluid environment, such as the human body.

### Description of the Prior Art

Osmotic devices manufactured in the form of tablets for delivering a drug to the gastrointestinal tract are known in United States Patent Nos. 3,845,770 and 3,916,899, both issued to inventors Felix Theeuwes and Takeru Higuchi. A typical device, disclosed in these patents, comprises a semipermeable wall that surrounds a compartment containing a drug. The wall is permeable to an external fluid and substantially impermeable to the passage of drug. There is a passageway through the wall for delivering drug from the system. The system releases drug by fluid being continuously imbibed through the wall into the compartment at a rate determined by the permeability of the wall and the osmotic pressure gradient across the wall to produce a solution containing soluble drug that is dispensed from the system over time.

In United States Patent No. 4,036,228, issued to Felix Theeuwes, the patentee discloses an osmotic device for delivering drugs that are hard to deliver, particularly drugs that are practically insoluble in aqueous-type fluids. The device is used by charging the device with an effervescent couple comprising an acidic component and a basic component. In operation when the device is in a fluid environment, the couple imbibes fluid into the device, thereby wetting the couple, causing it to react and produce in the device an effervescent solution. The effervescent solution creates a neutral condition in the device and it dispenses the drug from the device. The Theeuwes patent does not address itself to the delivery of drugs that exhibit limited solubilities in acidic environments, the use of only the basic components, and the production of effervescence in the environment of use.

### Disclosure of the Invention

The invention resides in the discovery that many anti-inflammatory drugs are difficult to deliver at controlled rates from an osmotic device. The anti-inflammatory drugs are poorly soluble in aqueous and acidic environments, such as the gastric fluid of the stomach, and these drugs on their delivery from an osmotic device precipitate on contact with the gastric fluid. This phenomenon occurs with precipitating drug accumulating at the exit of the passageway and on the exterior surface of the wall of the device. The precipitated drug impedes the flow of drug solution through the passageway of the osmotic device. This invention delivers the drug by mixing the drug with a carbon dioxide generating compound, which on its release from the device, and on contact with the acid in the gastric region reacts therewith and generates carbon dioxide gas. This physical activity and the gas dispense the drug in finely dispersed form from the device, while concomitantly keeping open the passageway of the device.

According to a first aspect of the invention therefore there is provided an osmotic device (10) adapted for the controlled delivery of a drug (14) to an acid fluid environment, said device comprising a semipermeable polymeric wall (11) permeable to the passage of fluid and substantially impermeable to the passage of drug, the wall surrounding and forming a compartment (12), a passageway (13) in the wall connecting the compartment with the exterior of the device for delivering drug from the device, characterized by the compartment containing a drug that exhibits limited solubility in the acid fluid environment, and a non-toxic basic compound capable of producing carbon dioxide on contact with the acid fluid environment, which compound is soluble in fluid imbibed into the compartment and exhibits an osmotic pressure gradient across the wall against the fluid of the environment, said compartment being substantially free of acid components capable of a carbon dioxide producing reaction with the basic compound.

According to a second aspect of the invention there is provided a method for substantially preventing the precipitation of a drug (14) in a passageway (13) of an osmotic device (10) when the device is present in an acidic environment, wherein said device comprises:

a) a shape retaining wall (11) formed of a semipermeable polymer selected from cellulose acetate, cellulose diacetate and cellulose triacetate, that is permeable to the passage of fluid from the environment of use, but which is substantially impermeable to the passage of drugs,

b) said wall (11) including passageway (13) therein to connect the compartment (12) with the exterior of the device to allow release of the drug from the compartment, said drug housed in said compartment being liable to precipitate in an acid environment, characterized in that said compartment also houses a non-toxic basic compound, said compound having a gas generating moiety which compound is soluble in, and reactive with, the fluid of the environment of use, said compartment being substantially free of acid components capable of a gas generating reaction with the basic compound, whereby said non-toxic compound on contact with the fluid environment produces a gas and thereby substantially prevents the precipitation of the drug in the passageway of the device.

### Brief Description of the Drawings

In the Drawings, which are included by way of illustration only

2

Figure 1, which is not drawn to scale, is an opened view of an osmotic device made with a semipermeable wall used to deliver drug;

Figure 2, which is not drawn to scale, is an opened view of an osmotic device made with a semipermeable microporous wall used to deliver drug;

Figure 3, depicts the release pattern of a poorly soluble drug from an osmotic device released without a gas generating compound; and

Figure 4, depicts the release pattern of a poorly soluble drug from an osmotic device released with a gas generating compound.

Description of Embodiments of the Invention By Way of Illustration

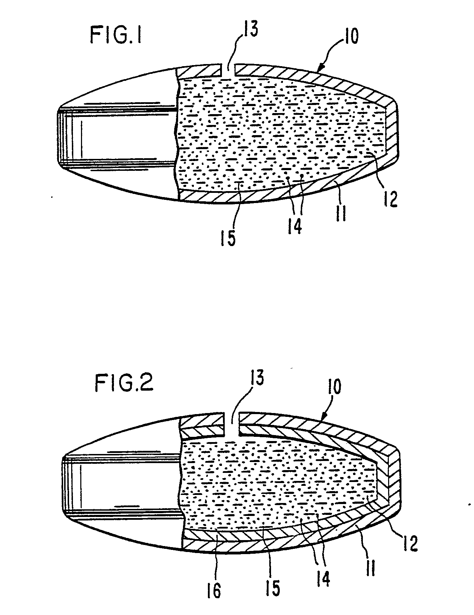

Figure 1 illustrates an osmotic device 10 used for delivering a drug. Osmotic device 10, is seen in opened view, which device 10 comprises a wall 11 formed of a semipermeable polymer that surrounds and forms a compartment 12. There is a passageway 13 through wall 11 connecting compartment 12 with the exterior of device 10 for delivering a drug 14 represented by dots, and a basic compound 15, represented by dashes, and having a carbon dioxide generating group from device 10.

Figure 2 illustrates an osmotic device 10 used for delivering a drug. Osmotic device 10, is seen in opened view, which device 10 comprises a wall 11 formed of a semipermeable polymer having laminated thereto a microporous lamina 16, facing the inside of compartment 12. In another embodiment, not shown, microporous lamina 16 can be on the outside surface of semipermeable wall 11. In Figure 2, device 10 has a passageway 13 through the semipermeable microporous laminate formed by 11 and 16, for connecting compartment 12 with the exterior of device 10 for delivering a drug 14 and a carbon dioxide generating compound 15 from device 10.

The semipermeable wall of the osmotic device 10 is formed of a semipermeable material that does not adversely affect the drug, the compound, or the patient. The semipermeable wall is formed of a polymeric material that is permeable to the passage of an exterior fluid, such as water and biological fluids, and it is essentially impermeable to the passage of drugs and solutes. The selectively permeable polymers useful for manufacturing the devices are represented by a member selected from the group consisting of cellulose acylate, cellulose diacylate, cellulose triacylate, cellulose acetate, cellulose diacetate, cellulose triacetate, polyamides, polyurethanes, and the like. Suitable semipermeable polymers for manufacturing osmotic devices are disclosed in United States Patent Nos. 3,845,770; 3,916,899; 4,008,719; 4,036,228; and 4,111,210. In the embodiment seen in Figure 2, the semipermeable wall can be a laminate comprising semipermeable lamina 11 in laminar arrangement with a microporous lamina 16. The semipermeable lamina 11 is formed of the above described polymers. The microporous lamina 16 has a plurality of micropores and interconnected micropaths for admitting fluid into the device. The microporous lamina can comprise the above polymers housing a pore former that is dissolved, or leached from the lamina, when the device is in operation in the environment of use. The pore formers are non-toxic and they do not react with the materials forming the wall. On their removal from the lamina, the paths formed therein fill with fluid, and these paths become a means for fluid to enter the device. Typical pore formers are represented by sodium chloride, potassium chloride, sorbitol, mannitol, polyethylene glycol, hydroxypropyl methylcellulose, hydroxypropyl butylcellulose, and the like. Osmotic devices having a laminated wall comprising a semipermeable lamina and a microporous lamina are disclosed in United States Patent No. 4,160,452.

The term "drugs" defines any substance that produces a local or a systemic beneficial effect in animals, avians, pisces and reptiles. The term animals includes primates, humans, household, sport and farm animals such as goats, cattle, horses, dogs, cats, and the like. The term animal also includes laboratory animals such as mice, rats, and guinea pigs. The drugs that can be delivered according to the invention include inorganic and organic drugs, such as central nervous system acting drugs, hypnotics, sedative, psychic energizers, tranquilizers, antidepressants, anticonvulsants, muscle relaxants, antiparkinson, anesthetics, anti-inflammatory, local anesthetics, antimalarials, hormones, sympathomimetics, diuretics, antiparasitics, neoplastics, hypoglycemics, ophthalmics, cardiacs and nutritionals.

The expression neutral relates to water and like biological fluids, and the expression acid relates to the stomach environment and the hydrochloric acid produced therein, the vaginal environment and the lactic acid produced therein, and other acid environments in an animal body. The phrase acidic body fluid as used herein denotes gastric fluid, vaginal fluid and other acid fluids in an animal body.

A specific group of drugs suitable for delivery to the above environments according to the invention are the acidic antiinflammatory drugs. The antiinflammatory drugs are represented by arylcarboxylic acid drugs, and enolic acid drugs. Examples of arylcarboxylic acid drugs include alclofenac or 4-allyloxy-3-chloro-phenylacetic acid; Aspirin (Registered Trademark) or acetylsalicylic acid; fenoprofen or dl-2-(3-phenoxyphenyl)propionic acid; flufenamic acid or 2-(3-trifluoromethylanilino)benzoic acid; ibuprofen or 2-(4-isobutylphenyl)propionic acid; indomethacin or 5-methoxy-2-methyl-1-(4'-chlorobenzoyl)-3-indole-acetic acid; ketoprofen or 2-(3-benzoylphenyl) propionic acid; metiazinic acid or 10-methyl-2-phenothiazinylacetic acid; naproxen or d-2-(6'-methoxy-2'-naphthyl)propionic acid; niflumic acid or 3-trifluoromethyl-2-phenylaminonicotinic acid; tolmetin or 1-methyl-5-p-tolyoyl-pyrrole-2-acetic acid; and· sulindac or cis-5-fluoro-2-methyl-1-[p-(methylsulfinyl)-benzylidene]indene-3-acetic acid. Examples of enolic acid drugs include azapropazone or 3-dimethylamino-7-methyl-1,2-(n-propyl- malonyl)-1,2-dihydro-1,2,4-benzotriazine; phenylbutazone or 3,5-dioxo-4-n-butyl-1,2-diphenyl-pryazolidine; prenazone or 4-prenyl-1,2-diphenyl-3,5-pyrazolidinedione, sudoxicam or 4-hydroxy-2-methyl-N-(2-thiazolyl)-2H-1,2-benzothiazine-3-carboxamide-1,1-dioxide; and the like. Other anti-inflammatory drugs include diclofenac or 2-[2,6-dichlorophenyl)amino] benzeneacetic acid; and peroxi-cam or 2H-1,2-benzothiazine-3-carboxamide.

Examples of other drugs that are practically insoluble or very slightly soluble in the environment of use that can be delivered by the device of the invention include diphenidol, meclizine hydrochloride, prochlorperazine maleate, anisindone, diphenadione, erythrityl tetranitrate, dizoxin, resperpine, acetazolamide, methazolamide, bendroflumethiazide, chlorpropamide, tolazamide, allopurinol, aluminum aspirin, salicylic acid, sodium salicylate, salicylamide, acetaminophen, acetophenetidin, colchicine, mefenamic acid, oxphenbutazone, zomepirac, methotrexate, acetyl sulfisoxazole, hydrocortisone, desoxycorticosterone acetate, cortisone acetate, triaminolone, 17-estradiol, 17-hydroxyprogesterone, 19-norprogesterone, prednisolone, progesterone, norethindrone acetate, norethynodrel, and the like.

The amount of drug present in a device will vary depending on the activity and the amount of drug to be administered to the host. Generally, the device will house from 0.5 mg to 3 g or more, with individual devices containing for example 25 mg, 50, 125 mg, 250 mg or 1.5 g. The drug can be in the device in various forms such as dispersion, granule, powder, pressed mass or film. Also, the drug can be mixed with a binder, diluent, dispersant, stabilizer or dye. The beneficial drugs, their solubilities, their present doses are known to the art in *Pharmaceutical Sciences*, by Remington, 15th Ed., 1975, published by the Mack Publishing Co. Easton, Penna; *The Drug, the Nurse, the Patient, Including Current Drug Handbook*, 1974—1976, by Falconer, et al., published by Saunder Company, Philadelphia, Penna; in *Physician Desk Reference*, 33rd Ed., 1979, published by Medical Economics Co., Oradell, N.J.; in *Ann. of Allergy*, Vol. 41, pages 75 to 77, 1979; in *Arzenim. Forsch.*, Vol. 25, pages 1629 to 1635, 1975; and in *J. Inter. Med. Res.*, Vol. 7, pages 335 to 338, 1979.

A suitable gas generating compound is a solid, basic compound that is pharmaceutically acceptable, and a) exhibits a concentration gradient across the semipermeable wall and imbibes fluid into the device, b) acts as a buffer and dissolves in fluid that enters the device forming a solution containing drug, c) raises outside of the device, the pH of the immediate surrounding area of the passageway high enough to lessen the rate of precipitation of the dug, and d) reacts, outside the device at the passageway environment interface, with the acid of the environment to produce carbon dioxide effervescence that directs drug away from the device in a finely, dispersed form. The basic compounds include non-toxic metal carbonate and bicarbonate salts, such as alkali metal carbonates and bicarbonates, the alkaline earth carbonates and bicarbonates, and mixtures thereof. The preferred compounds are those soluble in water and produce rapid effervescence on contact with the acid of the environment. A mixture of compounds with different degrees of solubility in water can be used with at least one compound being very soluble in water. Exemplary compounds include lithium carbonate, sodium carbonate, potassium carbonate, lithium bicarbonate, sodium bicarbonate, potassium bicarbonate, magnesium carbonate, calcium carbonate, magnesium bicarbonate and the like. Also useful gas generating compounds are ammonium carbonate, ammonium bicarbonate, ammonium sesquecarbonate, sodium sesquecarbonate and the like. These compounds, when dissolved in water, show a pH greater than 7, usually between 8 and 12. Optionally, it is often desirable to select the drug and the compound free of a common ion effect, so their respective solubilities in a fluid that enters the device are at their maximum. The amount of basic compound, or mixture thereof housed in the compartment generally is about 0.5 mg to 3 g, or more, and more preferably 25 mg to 750 mg. The compounds and their solubilities in water are disclosed in *The Handbook of Chemistry and Physics*, 48th Ed., 1968, published by the Chemical Rubber Co., Cleveland, Ohio.

The drug and the basic compound also can be used mixed with a binder and a lubricant. The drug and the compound are mixed in a water soluble binder, or in a water insoluble binder that releases the drug and compound on contact with water. Typical water soluble binders include poly(ethylene glycol), gelatin, agar, carboxycellulose, ethylmethyl-cellulose, poly(vinyl alcohol), poly(vinylpyrrolidone), water soluble starch derivatives, and the like. Typical lubricants include stearic acid, magnesium stearate, zinc stearate and the like. The amount of binder or lubricant used generally is about 0.1 mg to 150 mg, or more.

The osmotic devices of the invention are manufactured by standard techniques. For example, in one embodiment, the drug is mixed with the basic compound and other ingredients by ballmilling, calendering, stirring and pressing into a preselected shape. The material forming the wall of the device can be applied by dipping, molding or spraying the pressed mixture. One procedure for applying the wall is the air suspension technique. The air suspension technique can be used for manufacturing a wall formed of a single layer, or formed of a multiplicity of layers. The air suspension procedure is described in United States Patent No. 2,799,241; in *J. Am. Pharm. Assoc.*, Vol. 48, pages 451 to 459, 1959; and in *ibid*, Vol. 49, pages 82 to 84, 1960. An osmotic passageway or aperture through the wall is made by mechanical drilling, laser drilling, punching or cutting with die. A procedure for forming the passageway using a laser is described in United States Patent Nos. 3,916,899 and 4,088,864. Other standard manufacturing procedures are described in *Modern Plastic Encyclopedia*, Vol. 46, pages 62 to 70, 1969; in *Remington's Pharmaceutical Sciences*, 14th Ed., pages 1649 to 1698, 1970, published by Mack Publishing Co., Easton, Penna., and in *The Therapy and Practice of Industrial Pharmacy*, by Lachman, et al., pages 197 to 225, 1970, published by Lea & Febiger Co., Philadelphia, Penna.

The following examples illustrate the present invention.

### Example 1

An oral osmotic device for the delivery of a nonsteroid antiinflammatory drug sodium indomethacin was manufactured as follows: a drug composition was prepared for housing in the compartment of the device by thoroughly blending 105.2 mg of sodium indomethacin trihydrate, 142 mg of potassium bicarbonate, 5.0 mg of polyvinyl pyrrolidone, and 7.1 mg of stearic acid, and then compressing the homogenous blend into a pre-compartment forming drug formulation. Next, the compressed drug formulation was placed in an air suspension machine and coated with a microporous lamina forming composition. The microporous lamina composition comprised 45% by weight of cellulose acetate having an acetyl content of 39.8%, 27.5% by weight of hydroxypropyl methylcellulose, and 27.5% by weight of polyethylene glycol 4000. The lamina was formed from a methylene chloride-95% ethanol solvent (80:20 wt:wt). The microporous lamina was 0.128 mm (5 mil) thick.

Next, an exterior semipermeable lamina was laminated onto the microporous lamina, in the air suspension machine. The semipermeable lamina forming composition comprises 50% by weight of cellulose acetate having an acetyl content of 39.8% and 50% by weight of cellulose acetate having an acetyl content of 32%. The semipermeable lamina was applied from a solvent mixture comprising methylene chloride and 95% ethanol, 80:20 wt:wt. The systems were dried, and a 0.256 mm (10 mil) passageway was laser drilled through the laminated wall. The system releases indomethacin at the rate of 8 mg per hour. The device in operation, releases a solution that effervesces on contact with the acidic gastric fluid at the exit end of the passageway, producing carbon dioxide bubbles that disperse the drug in a fluffy state.

### Example 2

An oral osmotic device for the controlled and continuous delivery of indomethacin was made by following the general procedure described above. In the present device, the compartment housed a drug formulation comprising 56.4% potassium carbonate, 37.6% sodium indomethacin trihydrate, 3% Providone® and 3% stearic acid. The formulation after compressing had a diameter of 7.93 mm, an area of 1.6 cm$^2$ and a density of 1.65 g/ml. The device had a laminated wall comprising an interior microporous lamina consisting essentially of 45% by weight of cellulose acetate having an acetyl content of 39.8%, 45% by weight of sorbitol, and 10% by weight of polyethylene glycol 400. The lamina was applied from a solvent comprising methylene chloride-methanol-water, 62:35:3 by wt. A semipermeable lamina was laminated onto the microporous lamina, which semipermeable lamina consists of 50% by weight of cellulose acetate having an acetyl content of 39.8%, and 50% by weight of cellulose acetate having an acetyl content of 32%. The lamina was applied from a solvent consisting of methylene chloride and methanol, 80:20 by wt. The microporous forming lamina was 0.128 mm (5 mil) thick, and the semipermeable lamina 0.062 mm (2.4 mil) thick. The device had a 0.23 mm (9 mil) passageway and delivered indomethacin at the rate of 8 mg/hr. The device delivers the drug substantially free of rapid precipitation at the passageway environment interface, and on the wall of the device in the vicinity of the passageway.

### Example 3

The procedure of Example 2 was repeated with the conditions as described except that the microporous forming lamina was 0.0256 mm (1 mil) thick, the semipermeable lamina 0.069 mm (2.7 mil) thick, and the rate of release was 8 mg/hr.

### Example 4

The osmotic device of Examples 1 and 2 was manufactured in this example, wherein, (a) the microporous lamina was 5 mil thick, the semipermeable lamina was 3.4 mil thick, and the device has a release rate of 6 mg/hr, and (b) a device wherein the microporous lamina was 0.128 mm (5 mil) thick, the semipermeable lamina was 0.0435 mm) (1.7 mil) thick, and the system had a release rate of 12 mg/hr.

### Example 5

A series of oral osmotic devices for releasing an arylcarboxylic acid antiinflammatory drug in the gastrointestinal tract are prepared according to the invention, wherein the device houses from 40 to 250 mg of sodium indomethacin and more preferably from 85 to 125 mg of sodium indomethacin trihydrate the equivalent of 70 to 100 mg of indomethacin, from 50 to 300 mg of potassium bicarbonate and more preferably from 130 to 190 mg of potassium bicarbonate, 2 to 20 mg of binder, and more preferably 5 to 10 mg of binder, and 2 to 20 mg of lubricant, and more preferably 5 to 10 mgs. The device has an inner microporous forming lamina weighing 18 to 25 mg with a thickness of 0.10 to 0.16 mm, and an exterior semipermeable lamina weighing 6 to 20 mg with a thickness of 0.035 to 0.100 mm. The device has a passageway of 0.18 mm to 0.38 mm, and releases drug at the rate of 5 to

# 0 040 899

15 mg/hr.

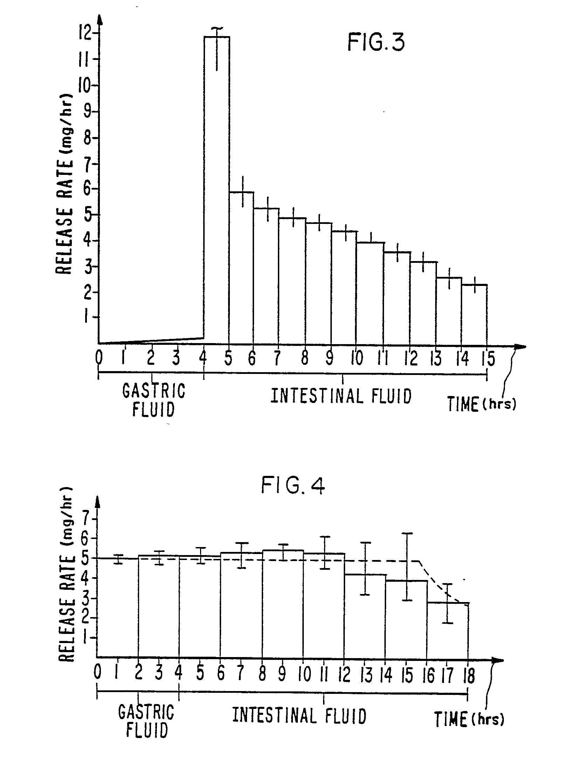

The unexpected benefits produced by the invention are seen in Figure 3 and Figure 4. Figure 1 shows the release of indomethacin from an osmotic device manufactured without a basic gas generating compound. The device releases the drug in the presence of an artificial gastric fluid containing hydrochloric acid, however, the drug precipitates onto the wall of the device and the exit port of the passageway, and it is therefore not observed in the fluid of the environment, which is analyzed on an hourly basis, as displayed in Figure 1. Figure 2 shows the release of indomethacin from an osmotic device manufactured with a basic gas generating compound. This device in operation releases drug in both an artificial intestinal fluid according to the invention.

## Claims

1. An osmotic device (10) adapted for the controlled delivery of a drug (14) to an acid fluid environment, said device comprising a semipermeable polymeric wall (11) permeable to the passage of fluid and substantially impermeable to the passage of drug, the wall surrounding and forming a compartment (12), a passageway (13) in the wall connecting the compartment with the exterior of the device for delivering drug from the device, characterized by the compartment containing a drug that exhibits limited solubility in the acid fluid environment, and a non-toxic basic compound capable of producing carbon dioxide on contact with the acid fluid environment, which compound is soluble in fluid imbibed into the compartment and exhibits an osmotic pressure gradient across the wall against the fluid of the environment, said compartment being substantially free of acid components capable of a carbon dioxide producing reaction with the basic compound.

2. The osmotic device for the controlled delivery of a drug according to Claim 1 wherein the basic compound having a carbon dioxide producing group is a pharmaceutically acceptable alkali metal carbonate, an alkali metal bicarbonate, an alkaline earth carbonate, or an alkaline earth bicarbonate.

3. The osmotic device for the controlled delivery of a drug according to Claim 1 or Claim 2 wherein the semipermeable polymeric wall has a microporous lamina (16) laminated thereto.

4. The osmotic device for the controlled delivery of a drug according to any preceding claim wherein the drug in the compartment is an anti-inflammatory arylcarboxylic acid drug.

5. The osmotic device for the controlled delivery of the drug according to any one of Claims 1 to 3 wherein the drug in the compartment is alclofenac, flufenamic acid, ibuprofen, indomethacin, ketoprofen, metiazinic acid, naproxen, niflumic acid, tolmetin, diclofenac, sulindac, azapropazone, phenylbutazone, prenazone, piroxicam, sudoxicam and sodium indomethacin trihydrate.

6. The osmotic device for the controlled delivery of a drug according to Claim 1 wherein the compound with a carbon dioxide producing group in the compartment is lithium carbonate, sodium carbonate, ammonium carbonate, potassium carbonate, lithium bicarbonate, sodium bicarbonate, ammonium bicarbonate, or potassium bicarbonate.

7. The osmotic device for delivery drug according to Claim 1 wherein the compartment contains the drug sodium indomethacin trihydrate and the carbon dioxide producing compound potassium bicarbonate.

8. The osmotic device for the delivering drug according to any preceding claim wherein the polymeric material forming the semipermeable wall is cellulose acylate, cellulose diacylate or cellulose triacylate.

9. The osmotic device for the controlled delivery of a drug according to any one of Claims 3 to 8 wherein the semipermeable wall has laminated thereto a microporous lamina formed of a cellulosic polymer containing a pore former.

10. A method for substantially preventing the precipitation of a drug (14) in a passageway (13) of an osmotic device (10) when the device is present in an acidic environment, wherein said device comprises:

a) a shape retaining wall (11) formed of a semipermeable polymer selected from cellulose acetate, cellulose diacetate and cellulose triacetate, that is permeable to the passage of fluid from the environment of use, but which is substantially impermeable to the passage of drugs,

b) said wall (11) including passageway (13) therein to connect the compartment (12) with the exterior of the device to allow release of the drug from the compartment, said drug housed in said compartment being liable to precipitate in an acid environment, characterized in that said compartment also houses a non-toxic basic compound, said compound having a gas generating moiety which compound is soluble in, and reactive with, the fluid of the environment of use, said compartment being substantially free of acid components capable of a gas generating reaction with the basic compound, whereby said non-toxic compound on contact with the fluid environment produces a gas and thereby substantially prevents the precipitation of the drug in the passageway of the device.

## Patentansprüche

1. Osmotische Vorrichtung (10), die geeignet ist zur gesteuerten Abgabe eines Arzneimittels (14) an eine saure flüssige Umgebung, wobei die Vorrichtung umfaßt eine semipermeable polymere Wand

6

(11), die für den Durchgang von Flüssigkeit durchlässig und für den Durchgang des Arzneimittels im wesentlichen undurchlässig ist, und die Wand eine Kammer (12) umgibt und bildet, einen Durchgang (13) in der Wand, der die Kammer mit dem Äußeren der Vorrichtung verbindet zur Abgabe des Arzneimittels aus der Vorrichtung, dadurch gekennzeichnet, daß die Kammer einen Wirkstoff enthält, der eine begrenzte Löslichkeit in der sauren flüssigen Umgebung besitzt, und eine nicht toxische basische Verbindung, die imstande ist, bei Berührung mit der sauren flüssigen Umgebung Kohlendioxid zu erzeugen, wobei die Verbindung, in der in die Kammer eingesaugten Flüssigkeit löslich ist und einen osmotischen Druckgradienten über die Wand gegenüber der Flüssigkeit der Umgebung erzeugt, wobei die Kammer im wesentlichen frei ist von sauren Bestandteilen, die imstande sind, mit der basischen Verbindung eine Reaktion unter Bildung von Kohlendioxid einzugehen.

2. Osmotische Vorrichtung zur gesteuerten Abgabe eines Arzneimittels nach Anspruch 1, wobei die basische Verbindung, die eine Kohlendioxid erzeugende Gruppe enthält, ein pharmazeutisch annehmbares Alkalicarbonat, ein Alkalibicarbonat, ein Erdalkalicarbonat oder ein Erdalkalibicarbonat ist.

3. Osmotische Vorrichtung zur gesteuerten Abgabe eines Arzneimittels nach Anspruch 1 oder Anspruch 2, wobei die semipermeable polymere Wand mit einer mikroporösen Schicht (16) laminiert ist.

4. Osmotische Vorrichtung zur gesteuerten Abgabe eines Arzneimittels nach einem der vorangehenden Ansprüche, wobei das Arzneimittel in der Kammer ein entzündungshemmendes Arylcarbonsäure-Arzneimittel ist.

5. Osmotische Vorrichtung zur gesteuerten Abgabe des Arzneimittels nach einem der Ansprüche 1 bis 3, wobei das Arzneimittel in der Kammer Alclofenac, Flufenaminsäure, Ibuprofen, Indomethacin, Ketoprofen, Metiazinsäure, Naproxen, Nifluminsäure, Tolmetin, Diclofenac, Sulindac, Azapropazon, Phenylbutazon, Prenazon, Piroxicam, Sudoxicam und Natrium-indomethacin-trihydrat ist.

6. Osmotische Vorrichtung zur gesteuerten Abgabe eines Arzneimittels nach Anspruch 1, wobei die Verbindung mit der Kohlendioxid bildenden Gruppe in der Kammer Lithium-carbonat, Natriumcarbonat, Ammonium-carbonat, Kalium-carbonat, Lithium-bicarbonat, Natrium-bicarbonat, Ammonium-bicarbonat oder Kalium-bicarbonat ist.

7. Osmotische Vorrichtung zur Abgabe von Arzneimittel nach Anspruch 1, wobei die Kammer das Arzneimittel Natrium-indomethacin-trihydrat und die Kohlendioxid bildende Verbindung Kaliumbicarbonat enthält.

8. Osmotische Vorrichtung zur Abgabe von Arzneimittel nach einem vorangehenden Ansprüche, wobei das polymere Material, das die semipermeable Wand bildet, Celluloseacylat, Cellulosediacylat oder Cellulosetriacylat ist.

9. Osmotische Vorrichtung zur gesteuerten Abgabe eines Arzneimittels nach einem der Ansprüche 3 bis 8, wobei die semipermeable Wand mit einer mikroporösen Schicht aus einem Cellulosepolymer, enthaltend einen Porenbildner, laminiert ist.

10. Verfahren zur weitgehenden Verhinderung der Ausfällung eines Arzneimittels (14) in einem Durchgang (13) osmotischen Vorrichtung (10), wenn die Vorrichtung in saurer Umgebung vorliegt, wobei die Vorrichtung umfaßt:

a) eine formerhaltende Wand (11) aus einem semipermeablen Polymer, ausgewählt aus Celluloseacetat, Cellulosediacetat und Cellulosetriacetat, das für den Durchgang der Flüssigkeit aus der Anwendungsumgebung durchlässig aber für den Durchgang von Arzneimitteln im wesentlichen undurchlässig ist,

b) die Wand (11), umfassend einen Durchgang (13) darin zur Verbindung der Kammer (12) mit dem Äußeren der Vorrichtung, um die Abgabe des Arzneimittels aus der Kammer zu ermöglichen, wobei das Arzneimittel in der Kammer dazu neigt, in saurer Umgebung auszufallen,

dadurch gekennzeichnet, daß die Kammer zusätzlich eine nicht-toxische basische Verbindung enthält, wobei die Verbindung eine gaserzeugende Gruppe enthält, und die Verbindung in der Flüssigkeit der Anwendungsumgebung löslich ist und mit ihr reagiert und wobei die Kammer im wesentlichen frei ist von sauren Bestandteilen, die imstande sind, mit der basisschen Verbindung eine gaserzeugende Reaktion einzugehen, wobei die nicht-toxische Verbindung bei Berührung mit der flüssigen Umgebung ein Gas erzeugt und dadurch die Ausfällung des Arzneimittels in dem Durchgang der Vorrichtung im wesentlichen verhindert.

**Revendications**

1. Dispositif osmotique (10) conçu pour la distribution réglée d'un médicament (14) dans un milieu fluide acide, ce dispositif comprenant une paroi polymère semi-perméable (11), perméable au passage de fluide et essentiellement imperméable au passage de médicament, cette paroi enveloppant et formant un compartiment (12), une voie de passage (13) prévue dans la paroi, et mettant en communication le compartiment avec l'extérieur du dispositif pour la distribution de médicament à partir de celui-ci, caractérisé en ce que le compartiment contient un médicament qui montre une solubilité limitée dans le milieu fluide acide, et un composé basique non toxique pouvant produire du dioxyde de carbone au contact avec le milieu fluide acide, ce composé étant soluble dans le fluide ayant pénétré dans le compartiment et montrant un gradient de pression osmotique à travers la paroi vis-à-vis du fluide du milieu, ce compartiment étant essentiellement exempt de composants acides pouvant réagir avec le composé basique en produisant du dioxyde de carbone.

2. Dispositif osmotique pour la distribution réglée d'un médicament suivant la revendication 1, caractérisé en ce que le composé basique comportant un groupe producteur de dioxyde de carbone est un carbonate de métal alcalin, un bicarbonate de métal alcalin, un carbonate alcalino-terreux ou un bicarbonate alcalinoterreux, pharmaceutiquement acceptables.

3. Dispositif osmotique pour la distribution réglée d'un médicament suivant l'une ou l'autre des revendications 1 et 2, caractérisé en ce qu'une lamelle microporeuse (16) est stratifiée sur la paroi polymère semi-perméable.

4. Dispositif osmotique pour la distribution réglée d'un médicament suivant l'une quelconque des revendications précédentes, caractérisé en ce que le médicament se trouvant dans le compartiment est un médicament anti-inflammatoire du type acide acrylcarboxylique.

5. Dispositif osmotique pour la distribution réglée d'un médicament suivant l'une quelconque des revendications 1 à 3, caractérisé en ce que le médicament se trouvant dans le compartiment est de l'alclofenac, de l'acide flufénamique, de l'ibuprofen, de l'indométhacine, du cetroprofen, de l'acide métiazinique, du naproxen, de l'acide niflumique, de la tolmétine, du diclofenac, du sulindac, de l'azapropazone, de la phénylbutazone, de la prénazone, du piroxicam, du sudoxicam ou du trihydrate d'indométhacine de sodium.

6. Dispositif osmotique pour la distribution réglée d'un médicament suivant la revendication 1, caractérisé en ce que le composé comportant un groupe producteur de dioxyde de carbone, se trouvant dans le compartiment, est le carbonate de lithium, le carbonate de sodium, le carbonate d'ammonium, le carbonate de potassium, le bicarbonate de lithium, le bicarbonate de sodium, le bicarbonate d'ammonium ou le bicarbonate de potassium.

7. Dispositif osmotique pour la distribution de médicament suivant la revendication 1, caractérisé en ce que le compartiment contient le médicament formé par le trihydrate d'indométhacine de sodium et en ce que le composé producteur de dioxyde de carbone est le bicarbonate de potassium.

8. Dispositif osmotique pour la distribution de médicament suivant l'une quelconque des revendications précédentes, caractérisé en ce que la matière polymère constituant la paroi semi-perméable est de l'acylate de cellulose, du diacylate de cellulose ou du triacylate de cellulose.

9. Dispositif osmotique pour la distribution réglée d'un médicament suivant l'une quelconque des revendications 3 à 8, caractérisé en ce qu'une lamelle microporeuse formée d'un polymère cellulosique contenant un agent formateur de pores est stratifiée sur la paroi semi-perméable.

10. Procédé pour empêcher pratiquement totalement la précipitation d'un médicament (14) dans une voie de passage (13) d'un dispositif osmotique (10), lorsque le dispositif est présent dans un milieu acide, dans lequel ce dispositif comprend:

a) une paroi de dimensions stables (11) formée d'un polymère semi-perméable choisi parmi l'acétate de cellulose, le diacétate de cellulose et le triacétate de cellulose, qui est perméable au passage de fluide provenant du milieu d'utilisation, mais qui est essentiellement imperméable au passage de médicaments,

b) cette paroi (11) comportant une voie de passage (13) mettant en communication le compartiment (12) avec l'extérieur du dispositif pour permettre la libération du médicament depuis le compartiment, ce médicament contenu dans ce compartiment étant susceptible de précipiter dans un milieu acide, caractérisé en ce que ledit compartiment contient également un composé basique non toxique, ce composé comportant un fragment producteur de gaz, lequel composé est soluble dans le fluide du milieu d'utilisation et réagit avec celui-ci, ce compartiment étant essentiellement exempt de composants acides pouvant réagir avec le composé basique en produisant du gaz, de sorte que le composé non toxique, au contact du milieu fluide, produit un gaz et, par conséquent, empêche pratiquement totalement la précipitation du médicament dans la voie de passage du dispositif.

8

FIG.1

FIG.2

0 040 899

FIG.3

RELEASE RATE (mg/hr)

GASTRIC FLUID     INTESTINAL FLUID     TIME (hrs)

FIG. 4

RELEASE RATE (mg/hr)

GASTRIC FLUID     INTESTINAL FLUID     TIME (hrs)

2